# EUROPEAN PATENT APPLICATION

(11) **EP 4 368 617 A1**
(43) Date of publication of application: **15.05.2024**
(21) Application number: 23816348.9
(22) Date of filing: 31.05.2023
(51) Int. Cl.: C07D 319/12, C08J 11/16, C08J 11/18, C07C 51/487, C07C 57/04, C07C 51/347, C08J 11/12, C08G 63/06

(54) **METHOD FOR PREPARING ACYLIC ACID AND/OR GLYCOLIDE**

(30) Priority: 31.05.2022 KR 20220066503; 31.05.2022 KR 20220066505; 26.09.2022 KR 20220121580; 26.09.2022 KR 20220121581; 30.05.2023 KR 20230069399
(71) Applicant: LG Chem, Ltd., Yeongdeungpo-gu Seoul 07336 (KR)
(72) Inventor: KIM, Si Min, Daejeon 34122 (KR); KANG, Donggyun, Daejeon 34122 (KR); JUNG, Woochul, Daejeon 34122 (KR)
(74) Representative: Goddar, Heinz J.
(86) International application number: PCT/KR2023/007414
(87) International publication number: WO 2023/234687

(57) **Abstract**

The present invention provides a method for producing acrylic acid and/or glycolide comprising thermally decomposing a hydroxyalkanoate copolymer in the presence of a catalyst containing tin.

## Description

### FIELD OF THE INVENTION

### CROSS-REFERENCE TO RELATED APPLICATION(S)

This application claims the benefit of Korean Patent Application No. 10-2022-0066505, filed on May 31, 2022, Korean Patent Application No. 10-2022-0066503, filed on May 31, 2022, Korean Patent Application No. 10-2022-0121581, filed on September 26, 2022, Korean Patent Application No. 10-2022-0121580, filed on September 26, 2022, and Korean Patent Application No. 10-2023-0069399, filed on May 30, 2023, the disclosures of which are incorporated herein by reference in their entirety.

The present invention relates to a method for producing acrylic acid and/or glycolide by thermally decomposing a hydroxyalkanoate copolymer.

### BACKGROUND OF THE INVENTION

Plastics are inexpensive and durable materials, which can be used to produce a variety of products that find use in a wide range of applications. As a consequence, the production of plastics has increased dramatically over the last decades. Moreover, 50% or more of such plastics are used for single-use disposable applications, such as packaging, agricultural films, disposable consumer articles, or for short-lived products that are discarded within a year of production. Because of the durability of the polymers, considerable quantities of plastics are piling up in landfill sites and in natural habitats worldwide, causing increasing environmental problems. Even degradable and biodegradable plastics may persist for decades depending on local environmental factors, such as levels of ultraviolet light exposure, temperature, presence of suitable microorganisms, etc.

Different solutions have been studied to reduce economic and environmental impacts correlated to the accumulation of plastic, from plastic degradation to plastic recycling.

As one example, polyethylene terephthalate (PET) is the most closed-loop recycled plastic (a system that processes and recycles waste from the production process). PET wastes (mainly bottles) are collected, sorted, pressed into bales, crushed, washed, chopped into flakes, melted and extruded in pellets and offered for sale. However, such plastic recycling methods are adapted to plastic articles containing only PET, and thus need a prior extensive sorting.

Another potential process for recycling plastic is chemical recycling allowing recovering the chemical constituents of the polymer. The resulting monomers may, after purification, be used to re-produce plastic articles. Thus, there is a need for a chemical recycling method for recycling it.

### SUMMARY OF THE INVENTION

### TECHNICAL PROBLEM

It is an object of the present invention to provide a method for producing acrylic acid and/or glycolide in high yield and high purity by thermally decomposing a hydroxyalkanoate copolymer.

### TECHNICAL SOLUTION

According to an embodiment of the present invention, there is provided a method for producing acrylic acid and/or glycolide comprising thermally decomposing a hydroxyalkanoate copolymer in the presence of a catalyst containing tin.

Now, a method for producing acrylic acid according to specific embodiments of the invention will be described in more detail.

Unless particularly mentioned herein, the term "including" or "comprising" refers to including some element (or component) without any limitation, and should not be construed as excluding addition of other elements (or components).

In addition, it should be understood that, unless steps included in a production method described herein are specified as being sequential or consecutive or otherwise stated, one step and another step included in the production method should not be construed as being limited to an order described herein. Therefore, it should be understood that an order of steps included in a production method can be changed within the range of understanding of those skilled in the art, and that, in this case, the incidental changes obvious to those skilled in the art are within the scope of the present invention.

As used herein, the term "polylactic acid prepolymer" refers to polylactic acid having a weight average molecular weight of 1,000 to 50,000 g/mol, which is obtained by oligomerizing lactic acid.

As used herein, the term "poly(3-hydroxypropionate) prepolymer" refers to poly(3-hydroxypropionate) having a weight average molecular weight of 1,000 to 50,000 g/mol, which is obtained by oligomerizing 3-hydroxypropionate.

Further, unless otherwise stated herein, the weight average molecular weight of polylactic acid prepolymer, poly(3-hydroxypropionate) prepolymer, and copolymer can be measured using a gel permeation chromatography(GPC). Specifically, the prepolymer or copolymer is dissolved in chloroform to a concentration of 2 mg/ml, then 20 µℓ is injected into GPC, and GPC analysis is performed at 40°C. At this time, chloroform is used as the mobile phase for GPC, the flow rate is 1.0 mL/min, and the column used is two Agilent Mixed-B units connected in series. RI Detector is used as a detector. The values of Mw can be obtained using a calibration curve formed using a polystyrene standard sample. Nine kinds of the polystyrene standard samples are used with the molecular weight of 2,000 g/mol, 10,000 g/mol, 30,000 g/mol, 70,000 g/mol, 200,000 g/mol, 700,000 g/mol, 2,000,000 g/mol, 4,000,000 g/mol, and 10,000,000 g/mol.

As used herein, the terms such as primary and secondary are used to describe various processes, and the terms are used only for the purpose of distinguishing one component (step) from another component (step).

According to one embodiment of the invention, there is provided a method for producing acrylic acid and/or glycolide comprising thermally decomposing a hydroxyalkanoate copolymer in the presence of a catalyst containing tin.

The present inventors have found that when a hydroxyalkanoate copolymer is thermally decomposed, acrylic acid and/or glycolide, which are recyclable monomers, can be recovered in high purity and high yield, and completed the present invention.

Also, the hydroxyalkanoate copolymer is thermally decomposed and recycled as a recyclable monomer, thereby being eco-friendly and economical, and the acrylic acid can be recycled into bio-superabsorbent polymer (SAP) or bioacrylate.

The method for producing acrylic acid and/or glycolide according to one embodiment may, before the thermally decomposing a hydroxyalkanoate copolymer in the presence of a catalyst containing tin, further comprise melting the hydroxyalkanoate copolymer. That is, the hydroxyalkanoate copolymer can be melted before the thermal decomposition, and this melting process can be performed in the presence of the catalyst containing tin.

Before thermal decomposition of the hydroxyalkanoate copolymer, volatile substances remaining due to the melting and/or impurities flowing in during recycling can be removed. Further, the mobility of the copolymer can be increased due to the melting, so that it can be easily added to a thermal decomposition reactor, and by utilizing this point, thermal decomposition can be continuously proceeded in the future.

The melting may be performed at a temperature of 150°C or more and 250°C or less. For example, the temperature during melting may be 150°C or more, 180°C or more, or 190°C or more, and 250°C or less, 230°C or less, or 220°C or less. If the melting temperature is too low, the hydroxyalkanoate copolymer may not be melted. If the melting temperature is too high, the hydroxyalkanoate copolymer is thermally decomposed without melting, which may reduce the recovery rate of the monomer, or large amounts of impurities may flow in because there is no process of removing impurities before thermal decomposition, and bumping or the like due to a sudden temperature rise may be generated.

On the other hand, the melting may be performed under a solvent-free condition. For example, when the melting is performed under a solvent-free condition, the hydroxyalkanoate copolymer may be melted in a state that is not dissolved in a solvent. For example, any other solvent in addition to the hydroxyalkanoate copolymer is not added to a reactor, and the temperature applied to the reactor can be transferred directly to the hydroxyalkanoate copolymer to melt it. When a solvent is added to the reactor, impurities may be formed in the process of thermally decomposing the hydroxyalkanoate copolymer, and a solvent removal process and an additional impurity removal process are required, which may complicate the process or require additional equipment. Further, side reactions may occur during the process of removing the solvent, which may result in a decrease in the recovery rate and purity of monomers. In addition, there is a disadvantage in that economic efficiency is lowered due to the use of additional solvents.

A method for producing acrylic acid and/or glycolide according to one embodiment may thermally decompose the melted hydroxyalkanoate copolymer in the presence of the catalyst containing tin.

On the other hand, the thermal decomposition may be performed at a temperature of 200°C or more and 400°C or less, and for example, the temperature may be 200°C or more, 205°C or more, 210°C or more, 215°C or more, and 400°C or less, 350°C or less. If the thermal decomposition temperature is too low, thermal decomposition of the hydroxyalkanoate copolymer may not occur, and if the thermal decomposition temperature is too high, the operating cost of the thermal decomposition process may increase, or large amounts of unexpected impurities may be generated.

Further, the thermal decomposition may be performed at a pressure of more than 0.01 torr and 50 torr or less, and for example, the pressure may be more than 0.01 torr, 0.05 torr or more, 0.1 torr or more, 0.5 torr or more, 1 torr or more, 2 torr or more, 4 torr or more, 5 torr or more, and 50 torr or less, 40 torr or less, 30 torr or less, or 20 torr or less, but is not limited thereto. As the thermal decomposition pressure is lower, the separation and recovery of the final produced bioacrylic acid can be facilitated.

The difference between the melting temperature and the thermal decomposition temperature may be 20°C or more and 130°C or less, 30°C or more and 120°C or less, 40°C or more and 110°C or less, 50°C or more and 100°C or less, or 60°C or more and 90°C or less. Because the difference between the melting temperature and the thermal decomposition temperature is small, that is, because the melting temperature is high and the thermal decomposition temperature is low, a continuous thermal decomposition process is facilitated, energy saving effects are achieved, and problems such as bumping due to a sudden temperature rise between melting and thermal decomposition processes can be prevented.

Moreover, the thermal decomposition can be performed under a solvent-free condition. For example, when the thermal decomposition is performed under a solvent-free condition, the hydroxyalkanoate copolymer may be thermally decomposed in a state that is not dissolved into a solvent. When a solvent is added to the reactor, impurities may be formed in the process of thermally decomposing the hydroxyalkanoate copolymer, and a solvent removal process and an additional impurity removal process are required, which may complicate the process or require additional equipment. Further, side reactions may occur during the process of removing the solvent, which may result in a decrease in the recovery rate and purity of monomers. Further, there is a disadvantage in that economic efficiency is lowered due to the use of additional solvents.

Further, the thermal decomposition may be performed in the presence of the catalyst containing tin. The catalyst containing tin remains in a solid phase even after the thermal decomposition process, which makes it easy to separate from the acrylic acid and/or glycolide that is finally produced, and thus the acrylic acid and/or glycolide can be recovered with high recovery rate and high purity.

The catalyst containing tin may be, for example, one or more selected from the group consisting of tin(II) 2-ethylhexanoate, tin(II) 2-methylhexanoate, tin(II) 2-propylhexanoate, dioctyltin dilaurate, dihexyltin dilaurate, dibutyltin dilaurate, dipropyltin dilaurate, diethyltin dilaurate, dimetyltin dilaurate, dibutyltin bis(lauryl mercaptide), dimethyltin bis(lauryl mercaptide), diethyltin bis(lauryl mercaptide), dipropyltin bis(lauryl mercaptide), dihexyltin bis(lauryl mercaptide), dioctyltin bis(lauryl mercaptide), dimethyltin bis(isooctylmaleate), diethyltin bis(isooctylmaleate), dipropyltin bis(isooctylmaleate), dibutyltin bis(isooctylmaleate), dihexyltin bis(isooctylmaleate) and dioctyltin bis(isooctylmaleate), but is not limited thereto.

The tin catalyst may be used in an amount of 0.0001 parts by weight or more, 0.0010 parts by weight or more, 0.0100 parts by weight or more, or 0.1000 parts by weight or more, and 10 parts by weight or less, 7 parts by weight or less, 5 parts by weight or less, 3 parts by weight or less, and 1 part by weight or less, based on 100 parts by weight of the hydroxyalkanoate copolymer.

The method for producing acrylic acid and/or glycolide may recycle biodegradable articles comprising the hydroxyalkanoate copolymer.

The hydroxyalkanoate copolymer is a biodegradable compound, and a biodegradable article containing the same is not particularly limited as long as it is a general article containing biodegradable plastic, but an example thereof may be a plastic bag, fiber, fabric, food container, toothbrush, film, fishing net, or packaging material. Further, the method for producing acrylic acid and/or glycolide according to one embodiment can thermally decompose a biodegradable article containing such a hydroxyalkanoate copolymer and recover monomers such as acrylic acid and glycolide, thereby recycling the biodegradable article. In addition, monomers such as acrylic acid and glycolide recovered through thermal decomposition can be polymerized to produce a biodegradable plastic, thereby recycling as biodegradable articles or the like.

In the method for producing acrylic acid and/or glycolide according to one embodiment, the hydroxyalkanoate copolymer may comprise two or more types of repeating units selected from the group consisting of the repeating units derived from 3-hydroxypropionic acid, the repeating units derived from lactic acid or lactide, and the repeating units derived from glycolic acid or glycolide. Further, in order to recover acrylic acid and/or glycolide in high purity and high yield, the hydroxyalkanoate copolymer may be 3-hydroxypropionate-lactide copolymer, glycolide-lactide copolymer, or 3-hydroxypropionate-glycolide copolymer.

Further, the hydroxyalkanoate copolymer may be a block copolymer containing two or more types of blocks selected from the group consisting of a block containing a repeating unit derived from 3-hydroxypropionic acid, a block containing a repeating unit derived from lactic acid or lactide, and a block containing a repeating unit derived from glycolic acid or glycolide. Further, in order to recover acrylic acid and/or glycolide in high purity and high yield, the hydroxyalkanoate copolymer may be 3-hydroxypropionate-lactide block copolymer, glycolide-lactide block copolymer, or 3-hydroxypropionate-glycolide block copolymer.

In the method for producing acrylic acid and/or glycolide according to one embodiment, the hydroxyalkanoate copolymer is the 3-hydroxypropionate-lactide copolymer.

The step of thermally decomposing a hydroxyalkanoate copolymer in the presence of a catalyst containing tin may comprises a step of by thermally decomposing the 3-hydroxypropionate-lactide copolymer in the presence of the catalyst containing tin to produce acrylic acid.

Further, as mentioned above, before the thermal decomposition, the 3-hydroxypropionate-lactide copolymer may be melted.

The 3-hydroxypropionate-lactide copolymer may be a block copolymer obtained by polymerizing a polylactic acid prepolymer and a poly(3-hydroxypropionate) prepolymer. The 3-hydroxypropionate-lactide copolymer exhibits excellent characteristic in a tensile strength and an elastic modulus that the polylactic acid prepolymer has, and also the poly(3-hydroxypropionate) prepolymer lowers the glass transition temperature(Tg), increases flexibility, and improves mechanical properties such as impact strength, thereby being able to prevent the characteristic (brittleness) of polylactic acid is that it has poor elongation and breaks easily.

The polylactic acid prepolymer may be produced by performing fermentation or polycondensation of lactic acid. The polylactic acid prepolymer may have a weight average molecular weight of 1,000 g/mol or more, or 5,000 g/mol or more, or 6,000 g/mol or more, or 8,000 g/mol or more, and 50,000 g/mol or less, or 30,000 g/mol or less. When it is desired to increase the crystallinity of the blocks containing repeating units derived from a polylactic acid prepolymer in the final produced block copolymer, it is preferred that the polylactic acid prepolymer has a high weight average molecular weight of more than 20,000 g/mol, or 22,000g/mol or more, or 23,000g/mol or more, or 25,000 g/mol or more, and 50,000 g/mol or less, or 30,000 g/mol or less, or 28,000 g/mol or less, or 26,000g/mol or less.

If the weight average molecular weight of the polylactic acid prepolymer is 20,000 g/mol or less, the crystals of the polymer are small, which makes it difficult to maintain the crystallinity of the polymer in the final produced block copolymer. If the weight average molecular weight of the polylactic acid prepolymer exceeds 50,000 g/mol, the rate of side reactions occurring within the prepolymer chain becomes faster than the reaction rate between the polylactic acid prepolymers during polymerization. On the other hand, 'lactic acid' as used herein refers to L-lactic acid, D-lactic acid, or a mixture thereof.

The poly(3-hydroxypropionate) prepolymer may be produced by performing fermentation or condensation polymerization of 3-hydroxypropionate. The weight average molecular weight of the poly(3-hydroxypropionate) prepolymer may be 1,000 g/mol or more, or 5,000 g/mol or more, or 8,000 g/mol or more, or 8,500 g/mol or more, and 50,000 g/mol or less, or 30,000 g/mol or less. When it is desired to increase the crystallinity of repeating units derived from poly(3-hydroxypropionate) prepolymer in the final produced block copolymer, it is preferable that the poly(3-hydroxypropionate) prepolymer has a high weight average molecular weight of more than 20,000 g/mol, or 22,000 g/mol or more, or 25,000 g/mol or more, and 50,000 g/mol or less, or 30,000 g/mol or less, or 28,000 g/mol or less.

As explained above for the polylactic acid prepolymer, if the weight average molecular weight of the poly(3-hydroxypropionate) prepolymer is 20,000 g/mol or less, the crystals of the polymer are small, which makes it difficult to maintain the crystallinity of the polymer in the final produced block copolymer. If the weight average molecular weight of the poly(3-hydroxypropionate) prepolymer exceeds 50,000 g/mol, the rate of side reactions occurring within the prepolymer chain becomes faster than the reaction rate between the poly(3-hydroxypropionate) prepolymers during polymerization.

That is, at least one of the polylactic acid prepolymer and the poly(3-hydroxypropionate) prepolymer may have a weight average molecular weight of more than 20,000 g/mol and 50,000 g/mol or less.

The 3-hydroxypropionate-lactide copolymer is a block copolymer obtained by polymerizing a polylactic acid prepolymer and a poly(3-hydroxypropionate) prepolymer, and the weight ratio of the polylactic acid prepolymer and the poly(3-hydroxypropionate) prepolymer in the block copolymer may be 95:5 to 50:50, 90:10 to 55:45, 90:10 to 60:40, 90: 10 to 70:30, or 90:10 to 80:20. If the poly(3-hydroxypropionate) prepolymer is contained in an excessively small amount relative to the polylactic acid prepolymer, the brittleness may increase, and if the poly(3-hydroxypropionate) prepolymer is contained in an excessively large amount relative to the polylactic acid prepolymer, the molecular weight may become low, and processability and heat resistance stability may deteriorate.

The 3-hydroxypropionate-lactide copolymer has a weight average molecular weight(Mw) of 50,000 to 300,000 g/mol, as measured using gel permeation chromatography(GPC). More specifically, the 3-hydroxypropionate-lactide copolymer has a weight average molecular weight of 50,000 g/mol or more, 70,000 g/mol or more, or 100,000 g/mol or more, and 300,000 g/mol or less, or 200,000 g/mol or less, or 150,000 g/mol or less. If the weight average molecular weight of the 3-hydroxypropionate-lactide is too small, the overall mechanical properties may be significantly reduced, and if the weight average molecular weight is too large, the process procedure may be difficult, and the processability and elongation may be lowered.

On the other hand, the lactic acid and 3-hydroxypropionate may be plastic and biodegradable compounds produced from renewable sources by microbial fermentation, and the block copolymer containing polylactic acid prepolymer and poly(3-hydroxypropionate) prepolymer formed by polymerizing them may also contain a large amount of bio raw materials while exhibiting eco-friendly property and biodegradability.

In addition, the acrylic acid produced by thermally decomposing a copolymer containing the bio raw material may also contain a large amount of bio raw material.

On the other hand, in the step of thermally decomposing the 3-hydroxypropionate-lactide copolymer to produce acrylic acid, the thermal decomposition may be performed at a temperature of 200°C or more and 350°C or less, for example, 200°C or more, 220°C or more, 240°C or more, 260°C or more, and 350°C or less, 330°C or less, 310°C or less, or 290°C or less. If the thermal decomposition temperature is too low, thermal decomposition of the hydroxyalkanoate-lactide copolymer may not occur, and if the thermal decomposition temperature is too high. large amounts of unexpected impurities may be generated.

Further, after the tin catalyst is added to the thermal decomposition process, an acid and a phosphonium salt may be added. As the tin catalyst is added to the thermal decomposition process, the 3-hydroxypropionate-lactide copolymer may be thermally decomposed to produce acrylic acid and lactide. After the acrylic acid and lactide are produced by adding the tin catalyst, when the acid and phosphonium salt are added, the lactide is converted to acrylic acid, and finally, only acrylic acid can be produced by the thermal decomposition process.

The acid can play the role of activating the carbonyl group of lactide, and can react with the phosphonium salt to produce acids such as bromic acid and hydrochloric acid, which can cause a ring-opening reaction of lactide, and substitute the hydroxy group with a halide group.

Further, the phosphonium salt reacts with the acid to produce acids such as bromic acid and hydrochloric acid, and substitutes the hydroxy group of the ring-opening reacted lactide with a halide group to finally produce acrylic acid.

For example, after adding the tin catalyst, acids and phosphonium salts may be added after the lapse of 30 minutes or more, 40 minutes or more, 50 minutes or more, or 1 hour or more, or after the lapse of 4 hours or less, 3 hours or less, or 2 hours or less. If the acid and phosphonium salt are added in a too short time after adding the tin catalyst, acid and phosphonium salts are added before the 3-hydroxypropionate-lactide copolymer is thermally decomposed to produce acrylic acid and lactide, which may generate other impurities other than acrylic acid. If the acid and phosphonium salt are added after too much time has elapsed after the tin catalyst is added, the material produced by the thermal decomposition process is exposed to strong heat for a long time, and side reactions may occur, thereby making it impossible to obtain the desired material.

The acid and the phosphonium salt may be added at the same time, or the phosphonium salt may be added after the acid is added, or the acid may be added after the phosphonium salt is added, but is not limited thereto.

The acid may be one or more selected from the group consisting of phosphoric acid, pyrophosphoric acid, metaphosphoric acid, polyphosphoric acid, phosphoric acid anhydride, phosphorous acid, hypophosphorous acid, sulfuric acid, nitric acid, silicic acid, hydrofluoric acid, boric acid, hydrochloric acid, perchloric acid, citric acid, tartaric acid, malic acid and salts of these acids.

The acid may be used in an amount of 0.0001 parts by weight or more, 0.0010 parts by weight or more, 0.0100 parts by weight or more, or 0.1000 parts by weight or more, and 10 parts by weight or less, 7 parts by weight or less, 5 parts by weight or less, 3 parts by weight or less, and 1 part by weight or less, based on 100 parts by weight of the 3-hydroxypropionate-lactide copolymer. If the amount of acid added is too small, it cannot function as an acid catalyst, and if the amount of acid added is too large, side reactions may occur.

The phosphonium salt may be a quaternary phosphonium salt. Also, the phosphonium salt may be, for example, one or more selected from the group consisting of tetrabutylphosphonium bromide, tetrabutylphosphonium chloride, tetraphenylphosphonium bromide, tetraphenylphosphonium chloride, dimethyldiphenylphosphonium bromide, dimethyldiphenylphosphonium chloride, methyltriphenoxyphosphonium bromide, methyltriphenoxyphosphonium chloride, hexadecyltributylphosphonium bromide, hexadecyltributylphosphonium chloride, octyltributylphosphonium bromide, octyltributylphosphonium chloride, tetradecyltrihexylphosphonium bromide, tetradecyltrihexylphosphonium chloride, tetrakis(hydroxymethyl)phosphonium bromide, tetrakis(hydroxymethyl)phosphonium chloride, tetraoctylphosphonium bromide, tetraoctylphosphonium chloride, tetramethylphosphonium bromide and tetramethylphosphonium chloride.

The phosphonium salt may be used in an amount of 0.0001 parts by weight or more, 0.0010 parts by weight or more, 0.0100 parts by weight or more, or 0.1000 parts by weight or more, and 10 parts by weight or less, 7 parts by weight or less, 5 parts by weight or less, 3 parts by weight or less, and 1 part by weight or less, based on 100 parts by weight of the 3-hydroxypropionate-lactide copolymer. If the amount of the phosphonium salt added is too small, acids such as hydrobromic acid and hydrochloric acid may not be produced, and conversion to acrylic acid may not occur, and if the amount of the phosphonium salt added is too large, there may be problems that side reactions occur and it is not economical.

Further, the weight ratio of the acid and phosphonium salt may be 1:0.1 to 10, 1:0.3 to 8, 1:0.5 to 6, 1:1.0 to 5, or 1:1.5 to 3. If the amount of phosphonium salt added is too small compared to the acid, acids such as bromic acid and hydrochloric acid may not be produced, and conversion to acrylic acid may not occur, and if the amount of phosphonium salt added relative to the acid is too large, there may be problems that side reactions may occur or it is not economical.

Moreover, the weight ratio of the tin catalyst and phosphonium may be 1:0.1 to 10, 1:0.3 to 8, 1:0.5 to 6, 1:1.0 to 5, or 1:1.5 to 3.

Through the thermal decomposition, acrylic acid can be recovered by distillation, at which time the acrylic acid recovery rate may be 30% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%. The recovery rate may be calculated on a weight basis.

The purity of the acrylic acid may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%.

In the method for producing acrylic acid and/or glycolide according to one embodiment, the hydroxyalkanoate copolymer is the 3-hydroxypropionate-lactide copolymer.

The step of thermally decomposing the hydroxyalkanoate copolymer in the presence of a catalyst containing tin may comprise:
a step of subjecting the 3-hydroxypropionate-lactide copolymer to primary thermal decomposition in the presence of the catalyst containing tin to produce lactide; and a step of subjecting the primary thermally decomposed hydroxyalkanoate-lactide copolymer to secondary thermal decomposition to produce acrylic acid.

Further, as mentioned above, before the thermal decomposition, the 3-hydroxypropionate-lactide copolymer may be melted. After the step of melting the 3-hydroxypropionate-lactide copolymer, the melted 3-hydroxypropionate-lactide copolymer may be subjected to primary thermal decomposition to produce lactide. Further, the primary thermally decomposed 3-hydroxypropionate-lactide copolymer may be subjected to secondary thermal decomposition to produce acrylic acid. At this time, the primary and secondary thermal decomposition can be performed under a solvent-free condition as described above.

The 3-hydroxypropionate-lactide copolymer is a block copolymer obtained by polymerizing polylactic acid prepolymer and poly(3-hydroxypropionate) prepolymer, and when this is subjected to primary thermal decomposition, lactide can be produced from the polylactic acid prepolymer, and when the primary thermally decomposed block copolymer is subjected to secondary thermal decomposition, acrylic acid can be produced from the poly(3-hydroxypropionate) prepolymer.

Meanwhile, in the step of subjecting the 3-hydroxypropionate-lactide copolymer to primary thermal decomposition in the presence of the catalyst containing tin to produce lactide, the primary thermal decomposition may be performed at a temperature of 200°C or more and 250°C or less, for example, 200°C or more, 210°C or more, 220°C or more, and 250°C or less, 240°C or less, and 230°C or less. If the primary thermal decomposition temperature is too low, thermal decomposition of the hydroxyalkanoate-lactide copolymer may not occur, and if the primary thermal decomposition temperature is too high, large amounts of unexpected impurities may be generated.

The primary thermal decomposition may be performed at a pressure of more than 1 torr and 50 torr or less, and for example, the pressure may be more than 1 torr, 2 torr or more, 4 torr or more, or 5 torr or more, and 50 torr or less, 40 torr or less, 30 torr or less, or 20 torr or less. If the primary thermal decomposition pressure is too low, it may be difficult to recover the produced lactide, and if the primary thermal decomposition pressure is too high, a large amount of energy is required, which may reduce economic efficiency. The primary thermal decomposition may be performed in the presence of a catalyst containing tin.

Moreover, after the primary thermal decomposition, the lactide can be separated by distillation under reduced pressure. For example, since the primary thermal decomposition is performed under reduced pressure conditions at a pressure exceeding 1 torr, the lactide produced at this time can be recovered by distillation under reduced pressure. Further, even if the primary thermal decomposition is not performed under reduced pressure conditions, the lactide can be recovered by distillation.

The 3-hydroxypropionate-lactide copolymer remaining after recovery of the lactide may be subjected to secondary thermal decomposition to produce acrylic acid.

On the other hand, in the step of subjecting the primary thermally decomposed hydroxyalkanoate-lactide copolymer to secondary thermal decomposition to produce acrylic acid, the secondary thermal decomposition may be performed at a temperature of 250°C or more and 300°C or less, for example, 250°C or more, 260°C or more, 270°C or more, or 280°C or more, and 300°C or less, or 290°C or less. If the secondary thermal decomposition temperature is too low, the primary thermally decomposed 3-hydroxypropionate-lactide copolymer may not be thermally decomposed, making it difficult to recover acrylic acid, and if the secondary thermal decomposition temperature is too high, large amounts of unexpected impurities may be generated.

The difference between the primary thermal decomposition temperature and the secondary thermal decomposition temperature may be 20°C or more and 100°C or less, for example, 20°C or more, 30°C or more, 50°C or more, 60°C or more, and 100°C or less, 90°C or less, 80°C or less, and 70°C or less. If the difference between the primary thermal decomposition temperature and the secondary thermal decomposition temperature is too small, it may be difficult to recover the acrylic acid, and if the difference between the primary thermal decomposition temperature and the secondary thermal decomposition temperature is too large, large amounts of unexpected impurities may be generated. Through the secondary thermal decomposition, acrylic acid can be recovered by distillation. At this time, the recovery rate of acrylic acid and lactide may be 30% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%. The recovery rate may be calculated on a weight basis.

The purity of the acrylic acid may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%.

The purity of the lactide may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%.

In the method for producing acrylic acid and/or glycolide according to one embodiment, the hydroxyalkanoate copolymer is a glycolide-lactide copolymer.

The step of thermally decomposing a hydroxyalkanoate copolymer in the presence of a catalyst containing tin may comprise a step of thermally decomposing the glycolide-lactide copolymer in the presence of the catalyst containing tin to produce lactide and glycolide.

Further, as mentioned above, before the thermal decomposition, the glycolide-lactide copolymer may be melted.

The glycolide-lactide copolymer is not particularly limited as long as it is a copolymer obtained by polymerizing a glycolide monomer and a lactide monomer, but may be, for example, a random copolymer obtained by polymerizing a glycolide monomer and a lactide monomer. Further, the glycolide-lactide copolymer may be a block copolymer including one or more polyglycolide blocks and one or more polylactide blocks. That is, the glycolide-lactide copolymer may be a block copolymer obtained by polymerizing a polylactide prepolymer and a polyglycolide prepolymer. As the glycolide-lactide copolymer contains the above-mentioned blocks, it can exhibit the eco-friendly property and biodegradability that polyglycolide and polylactide have.

The polylactide prepolymer may be produced by performing fermentation or polycondensation of lactic acid. The polylactide prepolymer may have a weight average molecular weight of 1,000 g/mol or more, or 5,000 g/mol or more, or 6,000 g/mol or more, or 8,000 g/mol or more, and 50,000 g/mol or less, or 30,000 g/mol or less. When it is desired to increase the crystallinity of the block containing repeating units derived from polylactide prepolymer in the final produced block copolymer, it is preferable that the polylactide prepolymer has a high weight average molecular weight of more than 20,000 g/mol, or 22,000 g/mol or more, or 23,000 g/mol or more, or 25,000 g/mol or more, and 50,000 g/mol or less, or 30,000 g/mol or less, or 28,000 g/mol or less, or 26,000 g/mol or less.

If the weight average molecular weight of the polylactide prepolymer is 20,000 g/mol or less, the crystals of the polymer are small, which makes it difficult to maintain the crystallinity of the polymer in the final produced block copolymer. If the weight average molecular weight of the polylactide prepolymer exceeds 50,000 g/mol, the rate of side reactions occurring within the prepolymer chain becomes faster than the reaction rate between the polylactide prepolymers during polymerization. On the other hand, 'lactic acid' as used herein refers to L-lactic acid, D-lactic acid, or a mixture thereof.

The polyglycolide prepolymer may be produced by performing fermentation or condensation polymerization of glycolide. The weight average molecular weight of the polyglycolide prepolymer may be 1,000 g/mol or more, or 5,000 g/mol or more, or 8,000 g/mol or more, or 8,500 g/mol or more, and 50,000 g/mol or less, or 30,000 g/mol or less. When it is desired to increase the crystallinity of repeating units derived from the polyglycolide prepolymer in the final produced block copolymer, it is preferable that the polyglycolide prepolymer has a high weight average molecular weight of more than 20,000 g/mol, or 22,000 g/mol or more, or 25,000 g/mol or more, and 50,000 g/mol or less, or 30,000 g/mol or less, or 28,000 g/mol or less.

As explained above for the polylactide prepolymer, if the weight average molecular weight of the polyglycolide prepolymer is 20,000 g/mol or less, the crystals of the polymer are small, which makes it difficult to maintain the crystallinity of the polymer in the final produced block copolymer. If the weight average molecular weight of the polyglycolide prepolymer exceeds 50,000 g/mol, the rate of side reactions occurring within the prepolymer chain becomes faster than the reaction rate between the polyglycolide prepolymers during polymerization.

That is, at least one of the polylactide prepolymer and the polyglycolide prepolymer may have a weight average molecular weight of more than 20,000 g/mol and 50,000 g/mol or less.

The glycolide-lactide copolymer is a block copolymer obtained by polymerizing a polylactide prepolymer and a polyglycolide prepolymer, and the weight ratio of the polylactide prepolymer and the polyglycolide prepolymer in the block copolymer may be 90:10 to 30:70, 80:20 to 40:60, or 75:25 to 50:50. If the polyglycolide prepolymer is contained in an excessively small amount relative to the polylactide prepolymer, the brittleness may increase, and if the polyglycolide prepolymer is contained in an excessively large amount relative to the polylactide prepolymer, the molecular weight may become low, and processability and heat resistance stability may deteriorate.

The glycolide-lactide copolymer has a weight average molecular weight (Mw) of 20,000 to 300,000 g/mol, as measured using a gel permeation chromatography (GPC). More specifically, the glycolide-lactide copolymer has a weight average molecular weight of 20,000 g/mol or more, 30,000 g/mol or more, 40,000 g/mol or more, 50,000 g/mol or more, 70,000 g/mol or more, or 100,000 g/mol or more, and 300,000 g/mol or less, or 200,000 g/mol or less, or 150,000g/mol or less. If the weight average molecular weight of the glycolide-lactide copolymer is too small, the overall mechanical properties may be significantly reduced, and if the weight average molecular weight is too large, the process procedure may be difficult, and the processability and elongation may be lowered.

In the method of producing lactide and glycolide according to one embodiment, the glycolide-lactide copolymer may be, for example, P2066 (lactide:glycolide = 65:35), P2191 (lactide:glycolide = 50:50), P1941 (lactide:glycolide = 75:25) or the like from Sigma-Aldrich. etc., or BD01128150 from BLD Pharm.

Further, after melting the glycolide-lactide copolymer, the melted glycolide-lactide copolymer may be thermally decomposed to produce lactide and glycolide. Similarly, the thermal decomposition can also be performed under a solvent-free condition.

On the other hand, in the step of thermally decomposing the glycolide-lactide copolymer in the presence of the catalyst containing tin to produce lactide and glycolide, the thermal decomposition may be performed at a temperature of 220°C or more and 350°C or less, and for example, the temperature may be 220°C or more, 250°C or more, or 290°C or more, and 350°C or less, 330°C or less, or 300°C or less. If the thermal decomposition temperature is too low, thermal decomposition of the glycolide-lactide copolymer may not occur, and if the thermal decomposition temperature is too high, large amounts of unexpected impurities may be generated.

The difference between the melting temperature and the thermal decomposition temperature may be 20°C or more and 150°C or less, and for example, the difference may be 20°C or more, 30°C or more, 50°C or more, or 60°C or more, and 150°C or less, 120°C or less, 100°C or less, or 70°C or less. If the difference between the melting temperature and the thermal decomposition temperature is too small, it may be difficult to recover the lactide and glycolide, and if the difference between the melting temperature and the thermal decomposition temperature is too large, large amounts of unexpected impurities may be generated.

Further, the thermal decomposition may be performed at a pressure of more than 1 torr and 50 torr or less, and for example, the pressure may be more than 1 torr, 2 torr or more, 4 torr or more, or 5 torr or more, and 50 torr or less, 40 torr or less, 30 torr or less, or 20 torr or less, but is not limited thereto. For example, if the thermal decomposition pressure is too low, it may be difficult to recover the produced lactide and glycolide, and if the thermal decomposition pressure is too high, a large amount of energy is required, which may reduce economic efficiency.

After the step of thermally decomposing the glycolide-lactide copolymer to produce lactide and glycolide, the lactide and glycolide can be recovered by distillation under reduced pressure.

For example, after the thermal decomposition process, the lactide and glycolide produced by reducing pressures to more than 1 torr can be recovered by distillation under reduced pressure. Further, lactide and glycolide can be recovered by distillation even under normal pressure conditions.

The recovery rates of the lactide and glycolide may be 30% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, respectively. The recovery rate may be calculated on a weight basis.

The purity of the lactide and glycolide may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%.

In the method for producing acrylic acid and/or glycolide according to one embodiment, the hydroxyalkanoate copolymer is the 3-hydroxypropionate-glycolide copolymer.

The step of thermally decomposing a hydroxyalkanoate copolymer in the presence of a catalyst containing tin may comprise a step of thermally decomposing the 3-hydroxypropionate-glycolide copolymer in the presence of a catalyst containing tin to produce acrylic acid and glycolide.

Further, as mentioned above, before the thermal decomposition, the 3-hydroxypropionate-glycolide copolymer may be melted. The melted 3-hydroxypropionate-glycolide copolymer may be thermally decomposed to produce acrylic acid and glycolide. At this time, the thermal decomposition can be performed under a solvent-free condition as mentioned above.

The 3-hydroxypropionate-glycolide copolymer includes a block containing a repeating unit derived from 3-hydroxypropionate and a block containing a repeating unit derived from glycolide, wherein these blocks are bonded via direct bonds, ester bonds, amide bonds, urethane bonds, or carbonate bonds, thereby compensating for the disadvantage of low elongation properties of biodegradable resins containing only polyglycolide. Further, these copolymers can have excellent biodegradability while complementing the mechanical properties of each homopolymer.

The 3-hydroxypropionate-glycolide copolymer includes a block containing a repeating unit derived from 3-hydroxypropionate represented by the following Chemical Formula 2, and a block containing a repeating unit derived from glycolide represented by the following Chemical Formula 3.

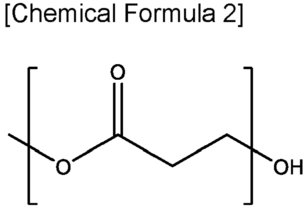

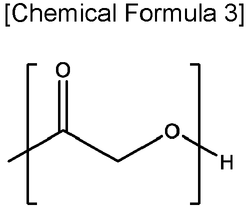

The repeating unit derived from 3-hydroxypropionate represented by Chemical Formula 2 has the advantage of having a glass transition temperature(Tg) as low as - 20°C while being excellent in mechanical properties, thereby exhibiting high elongation at break. Therefore, if such poly(3-hydroxypropionate) and polyglycolide are chemically combined to produce a block copolymer, it is possible to produce a biodegradable material with excellent mechanical properties.

Further, the repeating unit represented by Chemical Formula 2 and the repeating unit represented by Chemical Formula 3 may be linked through a direct bond, ester bond, amide bond, urethane bond, urea bond, or carbonate bond. For example, the hydroxyalkanoate-glycolide copolymer may be a block copolymer represented by the following Chemical Formula 1. 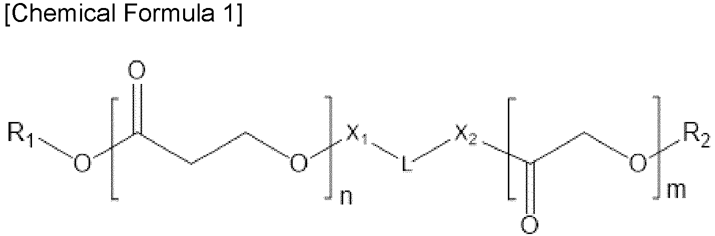 wherein in Chemical Formula 1,
R₁ and R₂ are each independently hydrogen, N, O, S, or a substituted or unsubstituted C₁₋₂₀ alkyl,
X₁ and X₂ are each independently a direct bond, -COO-, -NR'CO-, -(NR')(COO)-, -R'NCONR'-, or -OCOO-,
each R' is independently hydrogen, or a C₁₋₂₀ alkyl,
L is a direct bond; a substituted or unsubstituted C₁₋₁₀ alkylene; a substituted or unsubstituted C₆₋₆₀ arylene; or a substituted or unsubstituted C₂₋₆₀ heteroarylene containing one or more heteroatoms selected from the group consisting of N, O, and S, and
n and m may each independently be an integer from 1 to 10,000.

The n means the number of repetitions of repeating units derived from 3-hydroxypropionate, and as the n is introduced within the above range, physical properties such as elongation can be adjusted while maintaining the inherent physical properties of polyglycolide. Further, the m means the number of repetitions of the repeating unit derived from glycolide.

Furthermore, X₁, X₂, and L may be a direct bond.

The Chemical Formula 1 may be represented by the following Chemical Formula 1-1. 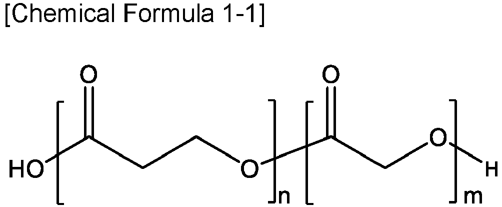 wherein, in Chemical Formula 1-1,
n and m may be as described above.

Preferably, n is 10 to 700, and m may be 10 to 700; n is 20 or more, 30 or more, 40 or more, 50 or more, or 60 or more, and 650 or less, 600 or less, 550 or less, 500 or less, or 450 or less; m is 20 or more, 30 or more, 40 or more, 50 or more, or 60 or more, and 650 or less, 600 or less, 550 or less, 500 or less, or 450 or less.

The 3-hydroxypropionate-glycolide copolymer may have a weight average molecular weight of 10,000 g/mol or more and 500,000 g/mol or less. For example, the weight average molecular weight of the copolymer is 12,000 g/mol or more, 15,000 g/mol or more, 20,000 g/mol or more, 25,000 g/mol or more, or 30,000 g/mol or more, and 480,000 g/mol or less, 460,000 g/mol or less, 440,000 g/mol or less, or 420,000 g/mol or less.

The 3-hydroxypropionate-glycolide copolymer may be a block copolymer obtained by ring-opening polymerization of a glycolide monomer in the presence of a poly(3-hydroxypropionate) initiator.

For example, the 3-hydroxypropionate-glycolide copolymer can be produced by a production method comprising: a step of preparing poly(3-hydroxypropionate) (step 1); and a step of preparing a block copolymer by ring-opening polymerization of a glycolide monomer in the presence of the poly(3-hydroxypropionate) initiator (step 2).

The step 1 is a step of preparing the poly(3-hydroxypropionate), wherein the poly(3-hydroxypropionate) refers to a homopolymer of 3-hydroxypropionic acid, and the one produced by adjusting the degree of polymerization in consideration of the above-mentioned ranges of n and m can be used.

The poly(3-hydroxypropionate) initiator may have a weight average molecular weight of 1,000 g/mol or more and 500,000 g/mol or less, 2,000 g/mol or more and 400,000 g/mol or less, 3,000 g/mol or more and 300,000 g/mol or less, 4,000 g/mol or more and 200,000 g/mol or less, 5,000 g/mol or more and 100,000 g/mol or less, or 10,000 g/mol or more and 90,000 g/mol or less.

The step 2 may be a step of performing a ring-opening polymerization of glycolide monomer using poly(3-hydroxypropionate) as an initiator. The step 2 can be carried out as bulk polymerization using substantially no solvent. At this time, using substantially no solvent may include an embodiment of using a small amount of solvent for dissolving the catalyst, for example, up to the embodiment of using less than 1 ml of solvent per 1kg of monomer used. As the step 2 proceeds by bulk polymerization, it becomes possible to omit the process for removing the solvent after polymerization, and decomposition or loss of the resin in such a solvent removal process can also be suppressed.

The weight ratio of the poly(3-hydroxypropionate) initiator and glycolide monomer may be 1:99 to 99:1, 5:95 to 90:10, 10:90 to 80:20, 15:85 to 70:30, or 20:80 to 50:50.

On the other hand, since the glycolide ring-opening polymerization reaction is involved, it can proceed in the presence of a glycolide ring-opening catalyst. For example, the ring-opening catalyst may be a catalyst represented by the following Chemical Formula 4.

[Chemical Formula 4] MA¹pA²₂₋ₚ

wherein, in Chemical Formula 4,
M is Al, Mg, Zn, Ca, Sn, Fe, Y, Sm, Lu, Ti or Zr,
p is an integer of 0 to 2, and
A¹ and A² may each independently be an alkoxy or carboxyl group.

For example, the catalyst represented by Chemical Formula 4 may be tin(II) 2-ethylhexanoate (Sn(Oct)₂).

The production of the 3-hydroxypropionate-glycolide copolymer may be performed at a temperature of 150 to 220°C for 5 minutes to 10 hours or for 10 minutes to 1 hour.

In the step of thermally decomposing the 3-hydroxypropionate-glycolide copolymer in the presence of a catalyst containing tin to produce acrylic acid and glycolide, the thermal decomposition may be performed at a temperature of 250 °C or more and 400 °C or less, wherein the temperature may be, for example, 250°C or more, 270°C or more, or 290°C or more, and 400°C or less, 370°C or less, and 330°C or less. If the thermal decomposition temperature is too low, thermal decomposition of the hydroxyalkanoate-glycolide copolymer may not occur, and if the thermal decomposition temperature is too high, large amounts of unexpected impurities may be generated.

The difference between the melting temperature and the thermal decomposition temperature may be 20°C or more and 150 °C or less, and for example, the difference may be 20°C or more, 30°C or more, 50°C or more, 60°C or more, 150°C or less, 120°C or less, 100°C or less, or 70°C or less. If the difference between the melting temperature and the thermal decomposition temperature is too small, it may be difficult to recover the acrylic acid and glycolide, and if the difference between the melting temperature and the thermal decomposition temperature is too large, large amounts of unexpected impurities may be generated.

Further, the thermal decomposition may be performed at a pressure of more than 1 torr and less than 50 torr, and for example, the pressure may be more than 1 torr, 2 torr or more, 4 torr or more, or 5 torr or more, and 50 torr or less, 40 torr or less, 30 torr or less, or 20 torr or less, but is not limited thereto. For example, if the thermal decomposition pressure is too low, it may be difficult to recover the produced acrylic acid and glycolide, and if the thermal decomposition pressure is too high, a large amount of energy is required, which may reduce economic efficiency.

After the step of thermally decomposing the hydroxyalkanoate-glycolide copolymer to produce acrylic acid and glycolide, the acrylic acid and glycolide can be recovered by distillation under reduced pressure.

For example, acrylic acid and glycolide produced by reducing the pressure to more than 1 torr after the thermal decomposition process can be recovered by distillation under reduced pressure. Further, acrylic acid and glycolide can be recovered by distillation even under normal pressure conditions.

The recovery rate of acrylic acid and glycolide may be 30% or more, 50% or more, 60% or more, 70% or more, 80% or more, oe 90% or more, and for example, the recovery rate may be 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, respectively. The recovery rate may be calculated on a weight basis.

The purity of acrylic acid and glycolide can be 50% or more and 60% or more, 70% or more, 80% or more, or 90% or more, and example, the purity may be 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, respectively.

### ADVANTAGEOUS EFFECTS

According to the present invention, it is possible to provide a method for producing acrylic acid and/or glycolide in which a hydroxyalkanoate copolymer is thermally decomposed in an eco-friendly and economical manner, and converted into recyclable acrylic acid and/or glycolide in high purity and high yield.

### DETAILED DESCRIPTION OF THE EMBODIMENTS

Hereinafter, the present invention will be described in more detail with reference to examples. However, the following examples are for illustrative purposes only, and are not intended to limit the scope of the present invention.

### Production Example 1: Production of 3-hydroxypropionate-lactide block copolymer

### (1) Production of polylactic acid prepolymer

25 g of an 85% L-lactic acid aqueous solution was added to a 100 ml Schlenk flask in an oil bath, and the pressure was reduced at 70°C and 50 mbar for 2 hours to remove moisture in L-lactic acid. Then, based on 100 parts by weight of lactate, 0.4 parts by weight of p-toluenesulfonic acid (p-TSA) and 0.1 parts by weight of SnCl₂ catalyst were added, respectively. A melt condensation polymerization reaction was performed at a temperature of 150°C for 12 hours. After the reaction was completed, the reaction product was dissolved in chloroform, and the extracted with methanol to obtain polylactic acid prepolymer (weight average molecular weight: 8,000 g/mol).

### (2) Production of poly(3-hydroxypropionate) prepolymer

25 ml of a 60% 3-hydroxypropionate aqueous solution was added to a 100 ml Schlenk flask in an oil bath, and moisture in 3-hydroxypropionate was removed at 50°C and 50 mbar for 3 hours, and then oligomerized at 70°C and 20 mbar for 2 hours. Then, 0.4 parts by weight of p-toluenesulfonic acid (p-TSA) catalyst based on 100 parts by weight of 3-hydroxypropionate was added to a reaction flask, and a melt condensation polymerization reaction was performed at a temperature of 110°C for 24 hours. After the reaction was completed, the reaction product was dissolved in chloroform, and the extracted with methanol to obtain poly(3-hydroxypropionate) prepolymer (weight average molecular weight: 26,000 g/mol).

### (3) Production of block copolymer

The polylactic acid prepolymer and the poly(3-hydroxypropionate) prepolymer were mixed at a weight ratio of 9:1 and added to a 100 ml Schlenk flask in an oil bath so that a total content was 30 g, to which 90 mg of p-toluenesulfonic acid (p-TSA) was then added, and annealed at 60°C for 3 hours. Then, a solid phase polymerization reaction was performed while mixing at 150°C and 0.5 mbar for 24 hours using an evaporator, thereby producing a 3-hydroxypropionate-lactide block copolymer (weight average molecular weight: 130,000 g/mol).

On the other hand, the weight average molecular weight of the polylactic acid prepolymer, poly(3-hydroxypropionate) prepolymer, and block copolymer was measured using a gel permeation chromatography (GPC).

### Production Example 2: Production of hydroxyalkanoate-glycolide copolymer

### (1) Production of poly(3-hydroxypropionate)

25 ml of a 60% 3-hydroxypropionate aqueous solution was added to a 100 ml Schlenk flask in an oil bath, and moisture in 3-hydroxypropionate was removed at 50°C and 50 mbar for 3 hours, and then oligomerized at 70°C and 20 mbar for 2 hours. Then, 0.4 parts by weight of p-toluenesulfonic acid (p-TSA) catalyst based on 100 parts by weight of 3-hydroxypropionate was added to a reaction flask, and a melt condensation polymerization reaction was performed at a temperature of 110°C for 24 hours. After the reaction was completed, the reaction product was dissolved in chloroform, and extracted with methanol to obtain poly(3-hydroxypropionate) (weight average molecular weight: 26,000 g/mol).

### (2) Production of block copolymer

20 g of glycolide, 5 g of poly(3-hydroxypropionate), and 20 mg of tin(II) 2-ethylhexanoate were added to a 500 mL Teflon-coated round flask, and vacuum-dried at room temperature for 4 hours by applying a vacuum sufficiently. Then, the flask was placed in an oil bath pre-heated to 130°C, the temperature of which was raised to 220°C, and then the ring-opening polymerization reaction was performed for 30 minutes. After the reaction was completed, residual monomers were removed through a devolatilization step of the product to obtain a final block copolymer (weight average molecular weight: 130,000 g/mol).

On the other hand, the weight average molecular weight of the poly(3-hydroxypropionate) and block copolymer was measured using a gel permeation chromatography (GPC).

### <Examples and Comparative Examples>

### Example 1

15 g of 3-hydroxypropionate-lactide block copolymer produced in Production Example 1 was added to a flask, and was fused by heating to 180°C. After confirming fusion, 0.03 ml of tin 2-ethylhexanoate was added thereto. Then, the reaction temperature was raised to 220°C, and the mixture was stirred for 1 hour. Then, 2.9 g of pyrophosphoric acid and 18 g of tetrabutylphosphonium bromide were added, and the mixture was stirred at 220°C for 1 hour. Then, the reaction product was heated to 290°C and then acrylic acid coming from distillation was recovered. After completion of the reaction, a total of 9 g of acrylic acid was recovered.

### Example 2

15 g of 3-hydroxypropionate-lactide block copolymer produced in Production Example 1 was added to a flask, and was fused by heating to 180°C. After confirming fusion, 0.03 ml of tin 2-ethylhexanoate was added thereto. Then, the reaction temperature was raised to 220°C, the reactor pressure was reduced to 5 torr to proceed a primary thermal decomposition reaction, and the distillated lactide was recovered. Then, the temperature inside the reactor was raised to 290°C, and the mixture was stirred to proceed the secondary thermal decomposition reaction, and then acrylic acid and lactide coming from distillation was recovered. After completion of the reaction, a total of 9 g of lactide and 2 g of acrylic acid were recovered, respectively.

### Example 3

15 g of 3-hydroxypropionate-lactide block copolymer produced in Production Example 1 was added to a flask, and was fused by heating to 180°C. After confirming fusion, 0.03 ml of tin 2-ethylhexanoate was added thereto. Then, the reaction temperature was raised to 220°C, and the mixture was stirred at that temperature for 1 hour. After stirring, the pressure inside the reactor was reduced to 5 torr to recover lactide. Then, the temperature inside the reactor was raised to 290°C to proceed a secondary thermal decomposition reaction, and then acrylic acid and lactide coming from distillation were recovered. After completion of the reaction, a total of 10 g of lactide and 2 g of acrylic acid were recovered, respectively.

### Example 4

15 g of glycolide-lactide copolymer (P1941, Sigma-Aldrich, lactide: glycolide = 75:25) was added to a flask, and was melted by heating to 220°C. After confirming melting, 0.03 ml of tin 2-ethylhexanoate was added thereto. Then, the reaction temperature was raised to 290°C, the reactor pressure was reduced to 5 torr, and then 3.0 g of glycolide (recovery rate: 79%) and 10.2 g of lactide (recovery rate: 91%) coming from distillation were recovered.

### Example 5

15 g of glycolide-lactide copolymer (P2191, Sigma-Aldrich, lactide: glycolide = 50:50) was added to a flask, and was melted by heating to 220°C. After confirming melting, 0.03 ml of tin 2-ethylhexanoate was added thereto. Then, the reaction temperature was raised to 290°C, the reactor pressure was reduced to 5 torr, and then 6.2 g of glycolide (recovery rate: 83%) and 6.5 g of lactide (recovery rate: 87%) coming from distillation were recovered.

### Example 6

15 g of the hydroxyalkanoate-glycolide copolymer produced in Production Example 2 was added to a flask, and was melted by heating to 220°C. After confirming melting, 0.03 ml of tin 2-ethylhexanoate was added thereto. Then, the reaction temperature was raised to 290°C, the reactor pressure was reduced to 5 torr, and then glycolide and acrylic acid coming from distillation were recovered.

### Comparative Example 1

10 g of lactide was added to a 100 ml 3-neck RB flask, and 2.9 g of solid pyrophosphoric acid and 18 g of tetrabutylphosphonium bromide were added at room temperature. Then, the inside temperature was raised to 220°C to proceed the reaction. After the reaction was completed, acrylic acid coming from distillation was recovered.

### Comparative Example 2

20 g of polylactic acid pellets were added, and fused by heating to 220°C. Then, 0.03 ml of tin 2-octylate was added thereto. The pressure was reduced to 5 torr at the same temperature, and then 16.3 g of lactide (recovery rate: 81.5%) coming from distillation was recovered.

### Comparative Example 3

5.0 g of poly(3-hydroxypropionic acid), 50 mg of pentamethyl diethylene triamine, and 10 mg of hydroquinone mono ethyl ether(MEHQ) was added to a flask and mixed with stirring. The reaction temperature was raised to 80°C, and the mixture was stirred for 2 hours. At this time, melting of poly(3-hydroxypropionic acid) was not observed. Then, the temperature inside the reaction was raised to 290°C, and then acrylic acid coming from distillation was recovered. After completion of the reaction, a total of 3.8 g of acrylic acid was recovered (recovery rate: 76%).

### Evaluation

### 1. Evaluation of recovery rate

The recovery rate (mol yield) of acrylic acid, lactide, and glycolide produced in Examples and Comparative Examples was calculated, and the results are shown in Table 1 below. If it was not recovered, it was indicated as '-'.

### 2. Evaluation of purity

The purity of acrylic acid, lactide, and glycolide produced in Examples and Comparative Examples was measured using ¹H NMR (400 MHz, CDCl₃), and the results are shown in Table 1 below.

**[Table 1]**

| | Examp le 1 | Examp le 2 | Examp le 3 | Examp le 4 | Examp le 5 | Examp le 6 | Comparati ve Example 1 | Comparati ve Example 2 | Comparati ve Example 3 |
|---|---|---|---|---|---|---|---|---|---|
| Recover y rate of acrylic acid (%) | 69 | 67 | 67 | - | - | 65 | 47 | - | 76 |
| Purity of acrylic acid (%) | 99 | 99 | 99 | - | - | 99 | 99 | - | 87 |
| Recover y rate of acrylic acid and lactide (acrylic acid:lacti de weight ratio) | - | 73 % (9:2) | 80% (10:2) | - | - | - | - | - | - |
| Recover y rate of lactide (%) | - | - | - | 91 | 87 | - | - | 82 | - |
| Purity of lactide (%) | - | - | - | > 98 | > 98 | - | - | > 98 | - |
| Recover y rate of glycolide (%) | - | - | - | 79 | 83 | 72 | - | - | - |
| Purity of glycolide (%) | - | - | - | > 98 | > 98 | 99 | - | - | - |

According to Table 1, it was confirmed that Example 1 has a high recovery rate and high purity of acrylic acid, and Examples 2 and 3 can recover lactide and acrylic acid in high purity and high yield, respectively. Further, it was confirmed that Examples 4 and 5 recover lactide with a recovery rate of 87% or more and glycolide with a recovery rate of 79% or more, and the purity of each of which exceeds 98%. Further, it was confirmed that Example 6 recovers acrylic acid and glycolide in high purity and high yield, respectively.

On the other hand, it was confirmed that Comparative Example 1 has a significantly low recovery rate of acrylic acid, and Comparative Example 2 recovers only lactide due to thermal decomposition of polylactic acid, but acrylic acid and/or glycolide cannot be recovered. Further, it was confirmed that in Comparative Example 3, the polymer was heated at 80°C for 2 hours, but the polymer was not melted. It was confirmed that When the polymer is thermally decomposed at 290°C, the pentamethyl diethylene triamine catalyst is also vaporized and mixed with the product acrylic acid, thereby significantly reducing the purity of acrylic acid.

## Claims

1. A method for producing acrylic acid and/or glycolide comprising thermally decomposing a hydroxyalkanoate copolymer in the presence of a catalyst containing tin.

2. The method for producing acrylic acid and/or glycolide according to claim 1, wherein:
before the thermally decomposing a hydroxyalkanoate copolymer in the presence of a catalyst containing tin,
the method further comprises melting the hydroxyalkanoate copolymer.

3. The method for producing acrylic acid and/or glycolide according to claim 2, wherein:
the melting is performed under a solvent-free condition.

4. The method for producing acrylic acid and/or glycolide according to claim 2, wherein:
the melting is performed at a temperature of 150°C or more and 250°C or less.

5. The method for producing acrylic acid and/or glycolide according to claim 1, wherein:
the thermal decomposition is performed under a solvent-free condition.

6. The method for producing acrylic acid and/or glycolide according to claim 1, wherein:
the hydroxyalkanoate copolymer comprises two or more types of repeating units selected from the group consisting of 3-hydroxypropionic acid-derived repeating units, lactic acid or lactide-derived repeating units, and glycolic acid or glycolide-derived repeating units.

7. The method for producing acrylic acid and/or glycolide according to claim 1, wherein:
the hydroxyalkanoate copolymer is a 3-hydroxypropionate-lactide copolymer, a glycolide-lactide copolymer or a 3-hydroxypropionate-glycolide copolymer.

8. The method for producing acrylic acid and/or glycolide according to claim 7, wherein:
the thermally decomposing a hydroxyalkanoate copolymer in the presence of a catalyst containing tin comprises,
thermally decomposing the 3-hydroxypropionate-lactide copolymer in the presence of the catalyst containing tin to produce acrylic acid.

9. The method for producing acrylic acid and/or glycolide according to claim 8, wherein:
the thermal decomposition is performed at a temperature of 200°C or more and 350°C or less.

10. The method for producing acrylic acid and/or glycolide according to claim 8, wherein:
after adding the catalyst containing tin, an acid and a phosphonium salt are added.

11. The method for producing acrylic acid and/or glycolide according to claim 7, wherein:
the thermally decomposing a hydroxyalkanoate copolymer in the presence of a catalyst containing tin comprises,
subjecting the 3-hydroxypropionate-lactide copolymer to primary thermal decomposition in the presence of the catalyst containing tin to produce lactide; and
subjecting the primary thermally decomposed hydroxyalkanoate-lactide copolymer to secondary thermal decomposition to produce the acrylic acid.

12. The method for producing acrylic acid and/or glycolide according to claim 11, wherein:
the primary thermal decomposition is performed in a temperature of 200°C or more and 250°C or less.

13. The method for producing acrylic acid and/or glycolide according to claim 11, wherein:
the secondary thermal decomposition is performed in a temperature of 250°C or more and 300°C or less.

14. The method for producing acrylic acid and/or glycolide according to claim 11, wherein:
a difference between the primary thermal decomposition temperature and the secondary thermal decomposition temperature is 20°C or more and 100°C or less.

15. The method for producing acrylic acid and/or glycolide according to claim 7, wherein:
the thermally decomposing a hydroxyalkanoate copolymer in the presence of a catalyst containing tin comprises,
thermally decomposing the glycolide-lactide copolymer in the presence of the catalyst containing tin to produce the lactide and the glycolide.

16. The method for producing acrylic acid and/or glycolide according to claim 15, wherein:
the thermal decomposition is performed in a temperature of 220°C or more and 350°C or less.

17. The method for producing acrylic acid and/or glycolide according to claim 7, wherein:
the thermally decomposing a hydroxyalkanoate copolymer in the presence of a catalyst containing tin comprises,
thermally decomposing the 3-hydroxypropionate-glycolide copolymer in the presence of the catalyst containing tin to produce the acrylic acid and the glycolide.

18. The method for producing acrylic acid and/or glycolide according to claim 17, wherein:
the thermal decomposition is performed in a temperature of 250°C or more and 400°C or less.

19. The method for producing acrylic acid and/or glycolide according to claim 1, wherein:
the method for producing acrylic acid and/or glycolide recycles biodegradable articles comprising the hydroxyalkanoate copolymer.
